# EUROPEAN PATENT APPLICATION

(11) **EP 1 974 736 A1**
(43) Date of publication of application: **01.10.2008**
(21) Application number: 07104789.8
(22) Date of filing: 23.03.2007
(51) Int. Cl.: A61K 36/185, A61K 36/24, A61K 36/42, A61K 36/47, A61K 36/48, A61K 36/9066, A61K 45/06, A61P 3/10

(54) **A composition for the treatment of diabetes mellitus**

(71) Applicant: Phyto Health Pharma B.V., 2289 CC Rijswijk (NL)
(72) Inventor: The designation of the inventor has not yet been filed
(74) Representative: van Loon, C.J.J.

(57) **Abstract**

The invention relates to the field of pharmacology. More specifically, the invention relates to a composition for the treatment of diabetes mellitus. Even more specifically, the invention relates to a herbal composition for treating the same. In one of its embodiments, the invention provides a composition comprising:
a) an anti hyperglycaemic agent
b) an anti inflammatory agent
c) an anti hyperlipidemic agent
d) an anti oxidant agent
e) a gastro-intestinal agent.

In a preferred embodiment, at least one of said agents is derived from a plant.

## Description

The invention relates to the field of pharmacology. More specifically, the invention relates to a composition for the treatment of diabetes mellitus. Even more specifically, the invention relates to a herbal composition for treating the same.

Diabetes mellitus is a chronic metabolic disorder characterized by a high blood glucose concentration (hyperglycemia), which is due to insulin deficiency or insulin resistance. It can be classified into type 1, type 2, "other types" or gestational diabetes (Table 1; National Diabetes Data Group, Diabetes 28:1039, 1979).

Acute signs and symptoms of uncontrolled diabetes are directly and immediately due to hyperglycaemia: glucosuria (glucose loss through urinating), polyuria (increased volume of urine), thirst, weight loss, fatigue, infection proneness, visual blurring (Harvey, Johns, Mc Kusick, Owens, Ross, 1988).

Diabetes mellitus is the most common disease associated with carbohydrate metabolism and is a major cause of disability and hospitalization. An estimated 30 million people worldwide had diabetes in 1985. By 1995, this number had shot up to 135 million. The latest WHO estimate for the number of people with diabetes, world-wide, in 2000 is 177 million. This will increase to at least 300 million by 2025 (WHO, Fact Sheet N° 236, Revised September 2002).

Chronic hyperglycaemia during diabetes causes glycation of body proteins that in turn leads to secondary complication affecting eyes, kidneys, nerves and arteries (Sharma et al., 1993). These may be delayed or lessened, by maintaining blood glucose values close to normal.

Diabetic Retinopathy is the leading cause of new blindness in people under 65 years of age in the USA (National Diabetes Advisory Board, 1987). Diabetes is a well-recognized risk factor for cataract and remains the major cause of blindness worldwide, accounting for half of the world's blind population (Thylefors, 1990). Currently surgical extraction of the involved lens is the sole treatment of cataract although effective, preventing or delaying the development remains the preferred approach.

Diabetes accelerates large vessel atherosclerosis, causing an increased incidence of peripheral vascular, coronary artery, and cerebrovascular disease (Collwell JA, 1985). About 60% of people with type II diabetes die of atherosclerotic cardiovascular disease; about 25% die of stroke (National Diabetes Advisory Board, 1987). Coexisting risk factors for atherosclerosis- especially smoking, hypertension, and increased LDL cholesterol or decreased HDL cholesterol add greatly to the risk of atherosclerosis in diabetes.

The number of deaths attributed to diabetes was previously considerably underestimated. A plausible figure is likely to be around 4 million deaths per year related to the presence of the disorder. This is about 9% of the global total. Many of these diabetes related deaths are from cardiovascular complications. Most of them are premature deaths when the people concerned are economically contributing to society. This situation is increasingly outstretching the health-care resources devoted to diabetes (WHO, Fact Sheet N° 236, Revised September 2002).

Because of its chronic nature, the severity of its complications and the means required to control them, diabetes is a costly disease, not only for the affected individual and his/her family, but also for the health authorities. Studies in India estimate that, for a low-income Indian family with an adult with diabetes, as much as 25% of family income may be devoted to diabetes care. For families in the USA with a child who has diabetes, the corresponding figure is 10%. The total health care costs of a person with diabetes in the USA are between twice and three times those for people without the condition. It was calculated, for example, that the cost of treating diabetes in the USA in 1997 was US$ 44 billion. In WHO's Western Pacific region a recent analysis of health care expenditure has shown that: 16% of hospital expenditure was on people with diabetes. Recent cost estimates, denied by similar methods to that quoted above for the USA, include those for Brazil (US$ 3.9 billion), Argentina (US$ 0.8 billion) and Mexico (US$ 2.0 billion). Each of these is an annual figure and is rising as diabetes prevalence increases (WHO, Fact Sheet N° 236, Revised September 2002).

Overall, direct health care costs of diabetes range from 2.5% to 15% annual health care budgets, depending on local diabetes prevalence and the sophistication of the treatment available. For most countries, the largest single item of diabetes expenditure is hospital admissions for the treatment of long-term complications, such as heart disease and stroke, kidney failure and foot problems. As the number of people with diabetes grows worldwide, the disease takes an ever-increasing proportion of national health care budgets. Introduction of cost-effective treatment strategies to reverse this trend are utmost necessary (WHO, Fact Sheet N° 236, Revised September 2002).

Since the largest single item of diabetes expenditure is hospital admissions for the treatment of long-term complications and since a diabetes epidemic is underway with many death related to long-term complications, the need for added benefits of new anti diabetic agents in the field of prevention or delay of long-term complications, is desired. It is also apparent that due to the side effects of the currently used drugs, there is a need for a safe agent with minimal adverse effects, which can be taken for longer durations.

In recent years the popularity of complementary medicine has increased considerably. Surveys conducted in Australia and USA indicate that almost 48,5% and 34% respondents had used at least one form of unconventional therapy including herbal medicine (Eisenberg et al., 1993; McLennan et al., 1996). Even WHO (1980) has suggested the evaluation of the potential of plants as effective therapeutic agents.

### Current treatment of diabetes

People with type 2 diabetes, especially those in the early stages, can manage their blood sugar through diet, weight loss, and physical activity. If this does not provide effective control, however, oral hypoglycaemic therapy and/or insulin are/is prescribed by doctors. There are five classes of medications available. Each has a different working mechanism (Table 3) and has its own side effects (Table 2). If one medication is not sufficient to lower the blood glucose levels to a desired extent, doctors may combine two or three diabetes treatment medications.

### Sulphonylureas

Sulphonylureas, such as Glucotrol® and Micronase®, are commonly prescribed medications for diabetes treatment. Sulphonylureas work by helping the body make insulin. They can be used alone or with other medications.

### Biguanides

Biguanides, such as Metformin (Glucophage®), help the body use insulin more effectively. It is often used by people who are overweight, since it also helps with weight control. It can be taken alone or with other medications.

### Alpha-glucosidase inhibitors

Alpha-glucosidase inhibitors, such as Precose® and Glyset®, work by slowing down the absorption of sugar in the digestive tract. They are often used in combination with other diabetes treatment medications.

### Thiazolidinediones

The overall action of thiozolidinediones is to make cells more sensitive to insulin. Medications include Avandia® and Actos®. Rezulin® was the first thiazolidinedione, but it was recently withdrawn from the market after it was determined it causes liver toxicity.

### Insulin

If oral medications do not control the blood sugar levels satisfactorily, insulin may be used for diabetes treatment. A person with type 2 diabetes needs insulin injections if his or her pancreas has stopped producing insulin altogether.

Although insulin therapy affords a tight and effective glycemic control, certain drawbacks such as oral ineffectiveness, short shelf live, requirement of constant refrigeration, parenteral therapy, and fatal hypoglycaemia in the event of excess dosage limit its usage. Moreover, pharmacotherapy with sulphonylureas and biguanides is also associated with side affects like hypoglycemia, weight gain, gastro-intestinal disturbances such as nausea, vomiting, diarrhea and lactate acidosis (regular renal function monitoring is, for example, recommended for metformin) (Rang and Dale, 1991). Thiazolidinediones (e.g. rosiglitazone), which reduce peripheral insulin resistance, are associated with weight gain due to water retention. Regular liver function monitoring is strongly recommended and thiazolidinediones are only prescribed as add on therapy.

The common anti diabetic drugs mostly have a single well-defined mechanism of action. There is a specific receptor system with which the active compound enters into a bond, leading to a specific cascade of reactions on molecular level and which ultimately leads to a specific effect on the glucose metabolism.

An exception to this is metformin, a member of the biguanides group of anti diabetic drugs. Its hypoglycemic effect is attributed to different actions (but all related to glucose in one way or the other) in contrast to most other anti diabetic drugs (sulphonylureas, thiazolidinediones) that have a single mechanism of action (Table 3).

In addition to the afore mentioned widely prescribed common anti diabetic drugs, some new drugs have been developed which only recently got a market approval. These new drugs and their classes are described below.

### Incretin Mimetics

Exenatide (Byetta®) exhibits many of the same effects as the human incretin hormone GLP-1. It has been shown to bind to and activate the known human GLP-1 receptor in vitro. Like GLP-1, Exenatide helps regulate glucose homeostasis through effects on multiple organs, including the pancreas, stomach, and liver:
- Increase in acute beta-cell response:
   o enhancement of glucose-dependent insulin secretion from beta cells in the pancreas;
   o restoration of first phase insulin response.
- Decrease in beta cell work load:
   o suppression of glucagon secretion from alpha cells in the pancreas, which leads to the reduction of hepatic glucose output from the liver;
   o slowing of gastric emptying, which allows for timely absorption of nutritients;
   o reduction of food intake.

Common side effects of Exenatide are: Acid or sour stomach, belching, diarrhoea, dizziness, feeling jittery, headache, heartburn, indigestion, nausea, stomach discomfort and vomiting.

### DPP-4 Inhibitors

GLP-1 is a 36 amino acid incretin hormone secreted from intestinal L-cells after the ingestion of a meal. The GLP-1 hormone in circulation acts at multiple levels to control glucose homeostasis. The active GLP-1 peptide is rapidly degraded by the DPP-4 enzyme which is expressed ubiquitously as a transmembrane ecto-enzyme and also found in the circulation in its soluble form. The inhibition of the enzyme DPP-4, leads to a larger circulating pool of the active form of GLP-1 which then confers multiple anti diabetes benefits. DPP4 inhibitors thus prolong the half-life of incretins resulting in increased insulin secretion, decreased glucagon secretion, delayed gastric emptying, and reduced food intake.

Januvia® is an example of a DPP-4 inhibitor. The most common side effects seen in Januvia® clinical studies were upper respiratory tract infection, sore throat, and diarrhoea.

### Inhaled Insulin

Exubera® consists of blisters containing human insulin inhalation powder, which are administered using the Exubera® Inhaler. A common side effect is hypoglycaemia. In clinical trials up to two years duration, patients treated with Exubera® demonstrated a greater decline in pulmonary function, specifically the forced expiratory volume in one second (FEV1) and the carbon monoxide diffusing capacity (DLCO), than comparator-treated patients. Because of the effect of Exubera® on pulmonary function, all patients should have spirometry (FEV1) assessed prior to initiating therapy with Exubera®. Assessment of pulmonary function (e.g., spirometry) is recommended after the first 6 months of therapy, and annually thereafter, even in the absence of pulmonary symptoms. Exubera® is contraindicated in patients who smoke or who have discontinued smoking less than 6 months prior to starting Exubera® therapy and in patients with underlying lung disease such as asthma and COPD.

Reference is further made to professional literature in which, current medical therapy and the side-effects, morbidity, epidemiology of diabetes mellitus and analysis of health care expenditure is reviewed in more detail.

As described above, current treatments are primarily directed to control glucose related processes in a diabetic patient. The present application describes medicaments that not only control glucose related processes, but that preferably also control oxidative stress, systemic inflammation and/or lipid abnormalities. In the case of diabetes mellitus, hyperglycemic control is important in long-term complications risk management, but the present application describes that it is not enough to treat hyperglycemia alone in the prevention of long term complications. Adequate focus on and treatment of proven risk factors as well as potential risk factors (oxidative stress, inflammation -sensitive plasma proteins) for diabetes associated coronary artery disease and diabetes associated renal disease is necessary. Integral treatment of diabetes mellitus offers hope of intervening to delay or prevent lesion progression and complications.

The present invention provides a composition that has a multifactor or multimodal mechanism. Hence, the present invention provides a composition that in respect of long term complication not only treats hyperglycemia, but also for example oxidative stress, inflammation and/or hyperlipidemia.

In a first embodiment, the invention provides a composition comprising:
a) an anti hyperglycaemic agent
b) an anti inflammatory agent
c) an anti hyperlipidemic agent
d) an anti oxidant agent
e) a gastro-intestinal agent.

As described in the experimental part herein, a composition of the invention is capable of at least in part reducing the blood glucose levels, i.e. the composition according to the invention comprises an anti hyperglycaemic agent.

Furthermore the herein described compositions also comprise anti-inflammatory properties. In a study by R. Yegnanarayan et al⁸ various models of inflammation were employed for testing the anti-inflammatory activity of extracts of *Curcuma Longa.* Carrageenin induced edema was taken as a prototype of exudative phase of inflammation and the granuloma pouch and cotton pellet tests were the methods involving proliferative phase of inflammation. The extracts of *C. Longa* showed significant anti-inflammatory activity in both exudative and proliferative inflammation and this activity was comparable to some extent with that of sodium salicylate, hydrocortisone, oxyphenbutazone and indomethacin.

The composition according to the invention is further capable of decreasing levels of triglycerides, VLDL (Very Low Density Lipoprotein), LDL (Low Density Lipoprotein), HDL (High Density Lipoprotein), and TC (Total Cholesterol), i.e. the composition according to the invention comprises an anti hyperlipidemic agent. Preferably, the level of at least one of said lipid components is significantly decreased. In an even more preferred embodiment, the levels of at least two or three lipidic components are significantly decreased.

It is thought that high blood glucose levels can cause the production of free radicals. An anti oxidant agent is capable of at least in part neutralising a free radical and hence an anti oxidant agent is beneficial in the treatment of diabetes mellitus (preferably type II).

A composition according to the invention further comprises a gastro-intestinal agent, i.e. an agent that allows proper absorption of the medication. This agent tones the digestion and improves at least partly the absorption of nutrients, which is often impaired in diabetes.

The above described composition is preferably used for the treatment of diabetes mellitus (preferably type II). Hence, the invention provides a composition for the treatment of diabetes mellitus (preferably type II) comprising: a) an anti hyperglycaemic agent, b) an anti inflammatory agent, c) an anti hyperlipidemic agent, d) an anti oxidant agent and e) a gastro-intestinal agent.

As will be discussed later on in more detail, a composition of the invention can be present in any form, for example as a powder, or as a solution or as a solution in which at least one of the agents is present as a aggregate or as a colloid.

A composition of the invention can comprise more than one anti hyperglycaemic agent, anti inflammatory agent, anti hyperlipidemic agent, anti oxidant agent or gastro-intestinal agent. In a preferred embodiment the invention provides a composition comprising at least one, two, three, four, five or even six anti hyperglycaemic agent(s), anti inflammatory agent(s), anti hyperlipidemic agent(s), anti oxidant agent(s) or gastro-intestinal agent(s) or any combination thereof.

The agents in the composition of the invention can be derived from a plant, but active agents of which the molecular structure is known can also be added as a synthetically agent. In a preferred embodiment, at least one agent of composition of the invention is derived from a plant, i.e. the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant. In yet another preferred embodiment, at least two, three or four (preferably, but not necessarily, different kinds of) agents are derived from a plant. In a preferred embodiment, all agents are derived from a plant.

If an agent in the composition of the invention is derived from a plant, it can be present as a raw whole or crushed plant or as an extract (standardized extract, quantified extract, other extract, alcoholic/aqueous extract or any combination thereof). Since active compounds can be found in higher concentrations in specific parts of medicinal plants, but are present too a lesser extent in other parts of the medicinal plant as well, all parts of the medicinal plants (roots, fruits, seeds, leaves, wood, or whole plants) can be processed.

Most medicinal plants have advantageous secondary actions in addition to their primary pharmacological action and hence, one plant that is used to derive a certain activity from can at the same time provide one, two or three (or even more) secondary activities. Table 7 for example describes for 8 different plants which primary and secondary activities are known for said plants. It should be clear to the skilled person that Table 7 does not provide a limiting listing of the activities of the mentioned plants. Plants and/or plant extracts can equally well be divided based on their anti atherosclerotic or cardioprotective activity (in stead of anti hyperlipidemic), immunostimulant or immunomodulatory activity (in stead of anti inflammatory) or long evity promoting or cytoprotective activity (in stead of anti oxidant).

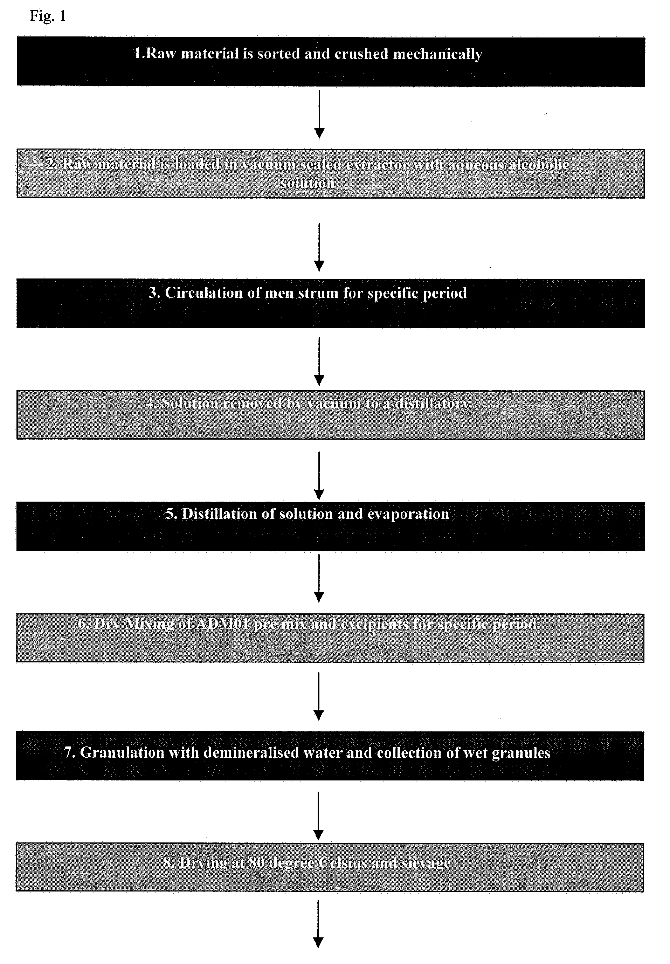

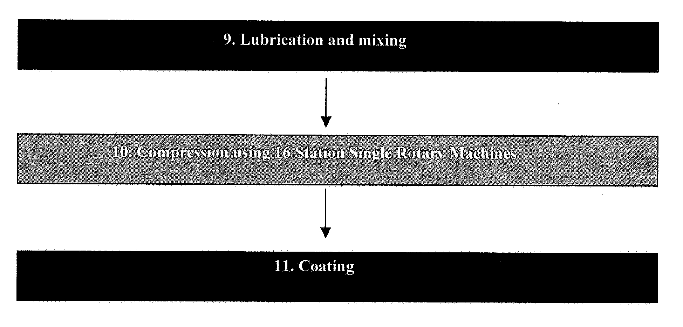

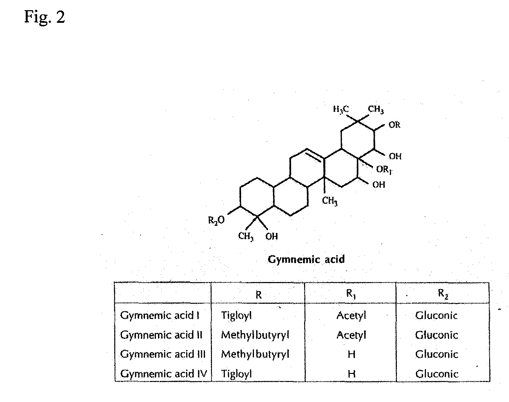

As described, active compounds from the medicinal plants with known molecular structures and pharmacological actions can be substituted by (synthetic) chemical compounds with similar molecular structures and pharmacological actions. Hence, in yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is provided as a synthetic counterpart. Examples of such (synthetic) chemical compounds are curcumin (1.7-Bis (4-hydroxy-3-mehtoxyphenyl)- 1.6-hepatadiene-3.5-dione) or gymnemic acid (see Figure 10). Curcumin is for example used to replace Curcuma Longa or an extract thereof and gymnemic acid is for example used to replace Gymnema Sylvestre or an extract thereof. Theoretically, all used extracts described herein can be replaced by a synthetic compound.

However, in a preferred embodiment, the invention uses medicinal plants or plant extracts in the preparation of a composition according to the invention. In one of the preferred embodiments, the invention provides a composition comprising:
a) an anti hyperglycaemic agent
b) an anti inflammatory agent
c) an anti hyperlipidemic agent
d) an anti oxidant agent
e) a gastro-intestinal agent, wherein said at least one agent is derived from a plant and wherein said at least one of agent is an anti hyperglycaemic agent. Hence, at least one plant or plant extract is used that has as a primary activity an anti hyperglycaemic activity. In a preferred embodiment, the anti hyperglycemic agent is derived from one or more plants from the group of Gymnema Sylvestre, Momordica Charantia, Trigonella Foenum Graecum, Curcuma Longa, Holarrhena Antidysenterica, Emblica Officinalis, Terminalia Belerica and Terminalia Chebula or a plant with comparable pharmacological action. Examples of plants with a comparable pharmacological action are for example provided in Table 8.

In yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti inflammatory agent. Hence, at least one plant or plant extract is used that has as a primary activity an anti inflammatory activity. In a preferred embodiment, the anti inflammatory agent is derived from one or more plants from the group of Curcuma Longa, Emblica Officinalis and Momordica Charantia or a plant with comparable pharmacological action. Examples of plants with a comparable pharmacological action are for example provided in Table 8.

In another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti hyperlipidemic agent. Hence, at least one plant or plant extract is used that has as a primary activity an anti hyperlipidemic activity. In a preferred embodiment, the anti hyperlipidemic agent is derived from one or more plants from the group of Emblica Officinalis, Trigonella Foenum Graecum, Terminalia Chebula, Terminalia Belerica, Curcuma Longa, Gymnema Sylvestre and Momordica Charantia or a plant with comparable pharmacological action. Examples of plants with a comparable pharmacological action are for example provided in Table 8.

In yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti oxidant agent. Hence, at least one plant or plant extract is used that has as a primary activity as an anti oxidant agent. In a preferred embodiment, the anti oxidant agent is derived from one or more plants from Curcuma Longa or Emblica Officinalis.
In another preferred embodiment, said anti oxidant agent is only present as a secondary activity. In a preferred embodiment, the anti oxidant agent is derived from one or more plants from the group of Terminalia Belerica and Terminalia Chebula or a plant with comparable pharmacological action. The amount of examples of plants with comparable pharmacological action (i.e. anti-oxidant) are vast and include, without being limiting, vegetables and fruits.

In a further preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an gastro-intestinal agent. Hence, at least one plant or plant extract is used that has as a primary activity a gastro-intestinal activity. In a preferred embodiment, the gastro-intestinal agent is derived from one or more plants from the group Holarrhena Antidysenterica and Curcuma Longa or a plant with comparable pharmacological action. Examples of plants with a comparable pharmacological action are for example provided in Table 8.

In yet another preferred embodiment, the invention provides a composition as described above, wherein the agents are present in the following amounts, based on total composition and preferably based on primary pharmacological action
a) 10 - 50 weight % of the anti hyperglycaemic agent
b) 10 - 30 weight % of the anti inflammatory agent
c) 10 - 50 weight % of the anti hyperlipidemic agent
d) 10 - 30weight % of the anti oxidant agent
e) 0.05 - 10 weight % of the gastro-intestinal agent
In yet another preferred embodiment, the provided ranges reflect the total activity (i.e. primary as well as secondary) of the mentioned agents.

In an even more preferred embodiment, the invention provides a composition as described above, wherein the agents are present in the following amounts, based on total composition and preferably based on primary pharmacological action
a) 20 - 40 weight % of the anti hyperglycaemic agent
b) 10 - 30 weight % of the anti inflammatory agent
c) 20 - 40 weight % of the anti hyperlipidemic agent
d) 10 - 30weight % of the anti oxidant agent
e) 0.05 - 10 weight % of the gastro-intestinal agent. In yet another preferred embodiment, the provided ranges reflect the total activity (i.e. primary as well as secondary) of the mentioned agents.

In a most preferred embodiment, the invention provides a composition as described above, wherein the agents are present in the following amounts, based on total composition and preferably based on primary pharmacological action
a) 30 weight % of the anti hyperglycaemic agent
b) 19 weight % of the anti inflammatory agent
c) 30 weight % of the anti hyperlipidemic agent
d) 19 weight % of the anti oxidant agent
e) 2 weight % of the gastro-intestinal agent. In yet another preferred embodiment, the provided ranges reflect the total activity (i.e. primary as well as secondary) of the mentioned agents. It is clear to the skilled person that minor deviations from the above mentioned percentages are within the scope of protection, for example a deviation of 10 % for each mentioned amounts for agents (a) to (d) and a deviation of 1 % for agent (e).

One of the preferred embodiments of the invention is a composition that is based on 8 plants and/or plant extracts. Preferably said composition comprises 3 plants and/or plant extracts that provide anti hyperglycaemic activity as a primary activity, 1 plant and/or plant extracts that provide anti-inflammatory activity as a primary activity, 3 plants and/or plant extracts that provide anti hyperlipidemic activity as a primary activity and 1 plant and/or plant extracts that provide a gastro intestinal improving effect as a primary activity. The invention therefore provides a composition comprising a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants. Examples of plants with a comparable pharmacological action are for example provided in Table 8. In a further preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, comprising a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants. Examples of plants with a comparable pharmacological action are for example provided in Table 8.

In a preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, comprising a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants, which comprises:
   a) 0.05 - 25 weight % of Curcuma Longa
   b) 0.05 - 25 weight % of Emblica Officinalis
   c) 0.05 - 25 weight % of Gymnema Sylvestre
   d) 0.05 - 25 weight % of Holarrhena Antidysenterica
   e) 0.05 - 25 weight % of Momordica Charantia
   f) 0.05 - 25 weight % of Terminalia Belerica
   g) 0.05 - 25 weight % of Terminalia Chebula
   h) 0.05 - 25 weight % of Trigonella Foenum Graecum

In a more preferred embodiment, all plants are present in a weight % range of 0.5-25 and even more preferably in a weight % range of 1-24 and most preferred in a weight % range of 2-24.

Each of the medicinal plants or extract components in each of the categories may be replaced by other medicinal plants with comparable (preferably comparable primary) pharmacological actions. To minimise the effect (if any) on the safety and efficacy profile, it is preferred to replace for example one, two, three or four of the mentioned medicinal plants with another plant with comparable (preferably comparable primary) pharmacological action. Examples of plants with a comparable pharmacological action are for example provided in Table 8.

Besides replacement compositions, a composition according to the invention can be expanded by the addition of additional (plant or plant derived) components. However, medicinal plants or extract components with comparable pharmacological actions may also be withdrawn from each category. Examples are provided in Table 9.

A composition according to the invention is preferably used in the preparation of a pharmaceutical and hence in a preferred embodiment, the invention provides a pharmaceutical composition comprising:
a) an anti hyperglycaemic agent
b) an anti inflammatory agent
c) an anti hyperlipidemic agent
d) an anti oxidant agent
e) a gastro-intestinal agent or any of the specific embodiments describes above. For example a pharmaceutical composition comprising a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants. Examples of plants with a comparable pharmacological action are for example provided in Table 8. A pharmaceutical of the invention can further comprise any pharmaceutical acceptable diluent, excipient, carrier or adjuvant.

Such a pharmaceutical may be presented in any pharmaceutical acceptable form, for example a solution, a suspension, a powder, a granule, a tablet or a capsule. A pharmaceutical of the invention is preferably used as a starting therapy which later on can be accompanied by other treatments or as an add on therapy, for example in combination with sulfonylurea, metformin or insulin. The described pharmaceutical is, for example, useful for patients who are on oral therapy, maximum tolerated therapy and insulin or insulin alone.

In yet another embodiment, the invention provides use of a composition comprising:
a) an anti hyperglycaemic agent
b) an anti inflammatory agent
c) an anti hyperlipidemic agent
d) an anti oxidant agent
e) a gastro-intestinal agent or any of the specific embodiments describes above, in the preparation of a medicament for the treatment of diabetes. Treatment preferably comprises alleviation of symptoms associated with diabetes. In a preferred embodiment, the invention provides the use of a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants in the preparation of a medicament for the treatment of diabetes. Examples of plants with a comparable pharmacological action are for example provided in Table 8.

In a preferred embodiment, said diabetes is diabetes mellitus type II. In a preferred embodiment said diabetes is uncomplicated diabetes.

Treatment according to the invention is especially useful to delay or prevent the development of long-term complications associated with diabetes mellitus. The invention therefore also provides use of a composition as described herein in the preparation of a medicament, to delay or prevent the development of long-term complications associated with diabetes mellitus. As a result, the health care costs for diabetes patients are reduced.

The invention further provides a method for treating a person in need thereof comprising administering an effective of amount of a composition as described herein to said person. In a preferred embodiment, said person is suffering from (symptoms associated with) diabetes.

The invention also provides a method for preparing any of the compositions described herein. Such a method for example comprises the steps of
- crushing a plant or a plant part or preparing an extract from a plant, wherein said plants are Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants
- mixing the obtained crushed plant or plant part or extracts
- optionally drying the obtained mixture
Now that it is clear that an effective long term complications risk reducing medicament preferably comprises a multifold mechanism of actions and focuses on co-treatment of proven and potential risk factors the same result can also be obtained by combining conventional medicaments for the treatment of hyperglycaemia, inflammation, hyperlipidemia and free radicals.

The invention will be explained in more detail in the following, non-limiting examples.

### Experimental part

### 1.1 Drug Profile

### Internal Drug Identification Code:

### ADKP

### Form and Presentation:

(1) biconvex tablet, yellow color coating, 500 mg or
(2) capsule shaped tablet, yellow color coating, 1062 mg

### Composition:

See Table 4 for amount and type of extract components per tablet of 500 mg. Content of Active Ingredients:
(1) 350 mg or (2) 675 mg
Excipients:
(1) Gum Acacia powder, Starch, Talc or (2) Microcrystalline cellulose, Ac-Di-Sol, Mg Stearate

### Indication:

Diabetes mellitus Type II

### Dosage and Directions for Use:

Dose1: 2 dd 2 tabs, total 2 gram
Dose2: 2 dd 3 tabs, total 3 gram
Dose3: 2 dd 4 tabs, total 4 gram

Dosage should be increased every 4 weeks based upon the fasting plasma glucose levels.

It is clear to the skilled person that for example the size, color, the total amount of active ingredients or dosage scheme etc of a tablet can be varied by the skilled person. The herein described example is by no means limiting.

### Contra Indications:

No data is available for use during pregnancy. No data is available for use in case of renal and hepatic failure. No data is available for use by children.

### Safety:

ADKP gives no habituation phenomena. If used as directed and within the ranges, ADKP appears to be a safe product. It does not cause hypoglycaemia in single use, however, hypoglycaemia can happen with concurrent use of conventional anti hypoglycaemic agents.

### 1.2 Efficacy

### In vitro studies

### Anti oxidant activity

In a 1,1-diphyenyl-picryl hydrazyl (DPPH) assay the antioxidant activity of ADKP and some of its extract components were evaluated against Curcumin as a standard anti oxidant. The antioxidant activity of ADKP appeared to be more than that of standard used and the drug showed either additive or synergistic effect of all the extract components to have the antioxidant activity.

It is thought that high blood glucose levels can cause the production of free radicals. Therefore, antioxidants are likely to benefit in diabetes treatment. The anti oxidant capacities of ADKP have been further studied in in vivo tests.

In streptozotocin treated rats (model mimics IDDM in humans), the level of Super Oxide Dismutase, a powerful enzyme and cellular anti-oxidant that acts as a super-scavenger of dangerous free-radicals, increased statistically significant (p= 0.007) in the ADKP treatment group against the diabetic control group, whereas SOD levels in the insulin treatment group did not significantly increase (p = 0.086).

### Insulinotropic activity

In an in vitro test using rat islets , the insulinotropic ability of ADKP was investigated against standard (tolbutamide) and placebo control.

There was a significant raise (p< 0.05) in rate of insulin secretion in ADKP treated rat islets in dose dependent manner at basal as well as stimulated glucose levels.

ADKP showed significant (p< 0.05) insulinotropic activity when compared with control, at least comparable with that of the standard drug tolbutamide (table 5).

### In vivo animal studies

In a study the effect of ADKP on blood glucose and lipid levels was investigated against a standard control group (glibenclamide) and diabetes control group (HFD) in experimental diabetes resembling NIDDM in humans, using the High Fructose Diet Model (HFD). Treatment lasted for 30 days.

The difference between treatments in changes of blood glucose levels (profiles) was statistically significant (p < 0.0005). There was a fall of blood glucose levels between day 1 and days 15 followed by a rise after about day 15.

When treatment groups were compared, statistically significant difference could be found in blood glucose levels between ADKP treatment groups and the HFD control group (p<0.0005) as well as in blood glucose levels between ADKP treatment groups and the glibenclamide treatment group (p=0.003).

The triglyceride levels significantly reduced when compared with HFD control group (p = 0.003), however TC (Total Cholesterol), LDL (Low Density Lipoprotein) and HDL (High Density Lipoprotein) levels did not significantly reduce (p> 0.05).

In another study streptozotocin was administered to the test animals, causing the destruction of the beta cells of pancreas. In rats a dose of STZ of 60mg/kg ip causes stable hyperglycemia by day 7 post administration. Generally, this model mimics human insulin dependent diabetes mellitus (IDDM). Groups were treated for 15 days with ADKP, ADKP + insulin and insulin.

STZ-pre treatment during 7 days significantly increased FPG-levels in all treatment groups (p = 0.030 to p = 0.048), but had no significant effect on insulin levels (p = 0.92).

Testing the diabetic control group against each single treatment for changes in FPG levels showed a statistically significant effect in all cases (Dunnett's posthoc tests, p = 0.001 to 0.037). Testing the diabetic control group against each single treatment for changes in insulin levels showed a statistically significant effect in all cases (Dunnett's posthoc tests, p < 0.0005 to p = 0.020), except against treatment group insulin 2.5' (p = 0.21).

ADKP significantly increased insulin levels and significantly decreased blood glucose levels in the 15 days of treatment. It was assumed that ADKP had no synergistic or antagonistic effect on insulin.

In the same study the effects of a single dose administration on fasting glucose levels was studied. Repeated measures ANOVA revealed a significant (p=0.002) fall of FPG-levels in the treated groups at 2 hrs (ADKP 30,8%, insulin 2.5 23,3% vs diabetic control 2%) and a relapse at 4 hrs. the effects of the 4 treatments (controls excluded) do not vary statistically significant p = 0.6).

Total Cholesterol levels were significantly reduced in the ADKP an other treatment groups (p < 0.0005) with respect to the diabetic control group. Differences between treatment groups were not significant (p>0.05). HDL, LDL and TG serum levels did not change significantly in either treatment group.

### Human studies

*Open label study*
*Randomized, placebo controlled, double blind study*
This experiment will be performed within the coming year.

### 1.3 Safety

### Pre clinical tests

In a toxicity study the behavioral, hematological and biochemical changes by once daily oral administration of ADKP, at three dose levels for 90 days to Sprague Dawley rats was studied and the toxic effects and potential target organs of toxicity of the test product was determined by following the OECD guideline 407, excepting gross morphological and histopathological studies. Two satellite groups (one control and other group with highest dose) were run additional 15 days to see reversibility of symptoms.

Parameters evaluated were mortality, morbidity, body weight, food intake, hematology, clinical biochemistry and urinary analysis. No deaths occurred except an unexplainable death on day 46 to a male rat treated with ADKP 500 mg/kg and no clinical signs were reported in any group. Body weights were unaffected by treatment.

In conclusion, administration of ADKP to the male and female Sprague Dawly rats orally for 90 days at 250, 500 and 1000 mg/kg do not seem to induce any toxicologically significant effects. Based on the data from this study, the No Observed Adverse Level (NOAEL) for male and female Sprague Dawley rats administered with ADKP is considered to be at least 1000 mg/kg.

### Clinical trials

Clinical studies with more than 50 patients who received ADKP treatment varying from 6-12 weeks reveal no serious safety issues.

No significant changes from baseline levels in vital parameters (pulse rate, systolic and diastolic blood pressure, respiratory rate and temperature) (p > 0.05) nor in laboratory tests (Hb, WBC, ESR, Creatinine, SGPT) (p>0.05), nor in ECG parameters have been registered.

No deaths or hypoglycaemic episodes have been reported.

### 1.4 Pharmacology

### 1.4.1 Mechanism of anti hyperglycaemic action

Pre clinical studies with extract components of ADKP show their hypoglycemic effect to be attributed to different actions (table 6):
1. stimulation of the release of endogenous insulin by the β-cells of the pancreas;
2. Improvement of the insulin-resistance;
3. Decrease in postprandial plasma glucose levels.

Therefore the ADKP mechanism of anti hyperglycaemic action can be termed as a multifactor or multimodal mechanism.

For some of the effects no specific receptor systems are identified and the mechanism is not completely understood (table 6).

### 1.4.2 Integrated treatment of the disease

Hyperglycemic control is important in diabetes treatment, but it is not enough to treat hyperglycemia alone.

Diabetes and impaired glucose tolerance have been associated with increased plasma concentrations of various inflammation-sensitive plasma proteins, including fibrinogen and C-reactive protein. Prospective studies have reported associations among various markers of inflammation and incidence of diabetes, and it has been proposed that inflammation has a causal role for the development of diabetes and diabetes associated vascular disease.

Diabetes constitutes a multiple source of free radicals, starting very early in the disease process and worsening over the course of disease. In view of the typical characteristics of diabetes, oxidative stress is expected to have a double impact on both metabolic and vascular functions. Therefore, antioxidants are likely to benefit in diabetes treatment.

High-density lipoprotein cholesterol (HDL) and high levels of total and VLDL triglycerides are characteristic lipid abnormalities in patients with type 2 diabetes. These Lipid abnormalities are a risk factor for coronary artery disease in patients with type 2 diabetes.

ADKP with its well balanced extract components intends an integrated treatment of the disease. Each of its extract components has either anti hyperglycaemic, anti inflammatory, anti oxidant or anti hyperlipidemic actions (Table 7).

### 1.5 Method of preparation

### Extraction

- Batches of medicinal plants are identified for authenticity by Quality Control (Q. C.) department.
- After receiving approval from the Q.C. department medicinal plant parts are sorted and crushed mechanically.
- The plant parts are then loaded in a stainless, vacuum-sealed extractor and an aqueous or alcoholic solution is added.
- The men strum is circulated for 4 hours and then removed by vacuum to a distillatory.
- The above process is repeated 4-5 times till the solution does not display any colour.
- The solution is distilled off and the sticky mass collected and dried to a powder.
- The extract is sent to Q.C. department.

### Dry Mixing

- After receiving approval from Q.C. department the standardized extracts of all extract components are weighed and mixed in prescribed quantities (ADKP pre mix).
- ADKP pre mix, Gum Acacia powder (10 %), starch (1 %) and talc (1 %) are weighed in required quantities depending upon the batch size and sieved through 80-mesh sieve.
- These ingredients are then loaded in a mass mixer and allowed to mix for 1 hour.

### Granulation

- After mixing for one hour, demineralised water is added and allowed to mix for 30 minutes.
- Wet granules are collected in steel trays.
- All trays are transferred in to tray drier.

### Drying

- Wet Granules are dried in tray drier at 80°C for 8 hours each on two consecutive days till all granules are dried completely. The temperature is checked thrice at the interval of 2 hours during the drying process.
- Dried granules are then passed through multi mill at a slow speed.
- Dried granules are passed through 40-mesh sieve.
- Moisture content of the dried granules is checked.
- Total weight of the dried granules is checked to ensure that it is +/- 5 % which is within permissible limits.

### Lubrication

- Dried granules are transferred in to previously weighed mass mixture.
- Weighed quantity of lubricants are passed through 100-mesh sieve and mixed in mass mixture at high speed for 1 hour.
- Lubricated granules are transferred to cleaned weighed container having P.V.C bag inside the container.
- Granules are weighed & reconciled the yield for +/- 5 % deviation which is within permissible limits.
- Samples of lubricated granules are withdrawn & sent to Q. C. department.
- After receiving approval from the Q. C. department granules are taken for compression.

### Compression

- Machine Used: 16 Station Single Rotary Machines
- Punches: Size: 11.065 mm; Shape: Semi Convex
- The compression is done using compression machine of above specification.

### Coating

- Coating is done using Pathan Coater.
- Coating mixture included: ponceu 4, titanium dioxide, carmoisine black, M.D.C and iso propyl alcohol in coating pan with the help of spray gun.
- Tablets are sent to the Q.C. department.

### Tables

**Table 1. Classification of diabetes mellitus**

| | |
|---|---|
| • Type 1: insulin-dependent diabetes mellitus (IDDM) | |
| • Type 2: non insulin-dependent diabetes mellitus (NIDDM) | |
| • Other types | |
| | - pancreatic disease |
| | - hormonal |
| | - drug or chemically induced |
| | - insulin receptor abnormalities |
| | - genetic syndromes |
| • Gestational diabetes | |

**Table 2: Side-effects**

| **Sulfonylurea** | |
|---|---|
| | hypoglycemia |
| | weight gain |
| | disturbance in liver function (rare) |
| | hypersensitivity reactions (rare) |
| | photosensitivity (rare) |
| | blood disorders (rare) |
| **Biguanides** | |
| | gastro-intestinal disturbances such as nausea, vomiting, diarrhoea lactate acidosis |
| | regular renal function monitoring is recommended |
| **Alpha-glucosidase inhibitors** | |
| | gastrointestinal problems including flatulence, diarrhea, and abdominal pain. |
| **Thiazolidinediones** | |
| | weight gain due to water retention |
| | regular liver function monitoring is strongly recommended |
| **Insulin** | |
| | Hypoglycaemia |
| | lipodystrophy |

as reported by H.P. Rang, M.M. Dale, 1991

**Table 3: Groups of anti diabetic drugs and their mechanisms of action**

| Group of Agents | Effect | Mechanism of Action |
|---|---|---|
| Sulfonylurea | insulin release from the beta cells is increased | Sulphonylureas bind to an ATP-dependant K⁺ channel on the cell membrane of pancreatic beta cells. This leads to an influx of potassium, a change in electric potential over the membrane, and opens voltage-gated Ca²⁺ channels. The rise in intracellular calcium leads to increased fusion of insulin granulae with the cell membrane, and therefore increased secretion of (pro) insulin. |
| | | |
| Thiazolidinedi ones | Insulin resistance is improved | Thiazolidinediones act by binding to PPARs (peroxisome proliferator-activated receptors), a group of receptors molecules inside the cell nucleus, specifically PPAR_{Y} (gamma). The normal ligands for these receptors are free fatty acids (FFAs) and eicosanoids. When activated, the receptor migrates to the DNA, activating transcription of a number of specific genes. By activating PPAR_{Y} insulin resistance is improved |
| | | |
| Alpha-glucosidase inhibitors | Postprandial glucose increase is reduced | Alpha-glucosidase inhibitors inhibit Apha-glucosidase in the intestinal wall which causes a delay in carbohydrate absorption. |
| | | |
| Biguanides | Insulin resistance is improved | The mechanism of Metformin's effect to reduce peripheral insulin resistance is not completely understood. |
| | | |
| | | The anti hyperglycemic action of Metformin has been attributed to a diminished intestinal absorption of carbohydrates, reduced gluconeogenesis, and increased peripheral glucose uptake. |

**Table 4: Amount and type of extract components per tablet of 500mg**

| # | Extract Component | Plant Part | Type of Extract | Quantity (mg) |
|---|---|---|---|---|
| 1. | Curcuma Longa | Root | Alcohol | 10 |
| 2. | Emblica Officinalis | Fruit | Aqueous | 80 |
| 3. | Gymnema Sylvestre | Leaf | Alcohol | 40 |
| 4. | Holarrhena | Seed | Aqueous | 10 |
| | Antidysenterica | | | |
| 5. | Momordica Charantia | Fruit | Alcohol | 80 |
| 6. | Terminalia Belerica | Fruit | Aqueous | 70 |
| 7. | Terminalia Chebula | Fruit | Aqueous | 50 |
| 8. | Trigonella Foenum | Seed | Aqueous | 10 |
| | Graecum | | | |

**Table 5: Effect of ADKP on amount of insulin release in rat islets at basal and stimulated glucose levels.**

| Name of the extract | Dose (in microgram) | Amount of insulin release at basal glucose levels (2 mM) picomoles/L | Amount of insulin during stimulated insulin release (glucose, 16 mM) picomoles/L |
|---|---|---|---|
| Control (rat islets only) | No drug | 63.45±4.59 | 170.09±8.90 |
| Tolbutamide (standard control) | 162 (1mM) | 107.08±15.35 | 239.48±11.43 |
| ADKP Extract dose (µg) | 10 µg | 91.01±1.70 | 184.27±5.28 |
| | 100 µg | 194.27±4.95 | 286.36±4.68 |
| | 1000 µg | 304.47±9.26 | 399.39±10.14 |

| | | | |
|---|---|---|---|
| *- insulinotropic ability of extracts are expressed as mean*±*SEM* *- Assay is repeated 3 times and average of 3 values are reported* | | | |

**Table 6: Extract Components ADKP, Mechanism of Action**

| Effect | Mechanism of Action (Extract Component) |
|---|---|
| Stimulation of insulin- secreting beta cells | ADKP stimulates beta cells functioning: |
| | -Increase of C-peptide level/enhanced endogenous insulin production (GS) |
| | -Renewal/recovery of b-cells (MC,GS) |
| | |
| Increase of glucose uptake and utilization in tissues | ADKP increases peripheral glucose uptake and incorporation of glucose into glycogen and protein in the liver, kidney and muscle and reduces gluconeogenesis by the liver (MC). |
| | |
| | ADKP increases the activities of the enzymes affording the utilisation of glucose by insulin dependent pathways: it controls phosphorylase levels, gluconeogenic enzymes and sorbitol dehydrogenase (GS). |
| | |
| | Increase in GLUT4 protein content in muscle plasma membrane (MC) |
| | |
| | Decrease in insulin resistance as measured by HOMA (TFG) |
| | |
| Postprandial glucose increase is reduced | ADKP Delays carbohydrate absorption: |
| | -Dietary fibre and delayed gastric emptying (TFG) |
| | -Inhibition of glucose uptake incorporated with Na+ entry processes (GS) |

**Table 7: Primary and Secondary Actions**

| *#* | *Herb* | *Primary action* | *Secondary action* |
|---|---|---|---|
| 1. | Curcuma Longa | II, V | I, III, IV |
| 2. | Emblica Officinalis | III, V | I, II |
| 3. | Gymnema Sylvestre | I | III |
| 4. | Holarrhena Antidysenterica | IV | I |
| 5. | Momordica Charantia | I | II, III |
| 6. | Terminalia Belerica | III | I, V |
| 7. | Terminalia Chebula | III | I, V |
| 8. | Trigonella Foenumgraecum | I | III |

| | | | |
|---|---|---|---|
| ** Notations* *I anti hyperglycemic effect* *II ant -inflammatory effect* *III anti hyperlipidemic effect* *IV gastro-intestinal improving effect* *V anti oxidant effect* | | | |

**Table 8 List of medicinal plants with comparable pharmacologic actions**

| LIST OF ANTI-HYPERGLYCEMIC MEDICINAL PLANTS |
|---|
| **Latin names** |
| Allium cepa |
| Allium sativum |
| Asparagus racemosa |
| Azadirachta indica |
| Pterocarpus marsupium |
| Tinospora cordifolia |
| Coccinia indica |
| Lagerstroemia speciosa |
| Ficus religiosa |
| Inula racemosa |
| Aegle marmelos |
| Ocimum sanctum |
| Eugenia jambolina |
| Phyllanthus niruri |
| Moringa oleifera |
| Ficus glomerata |
| Ficus benghalensis |
| Cinnamomum tamala |
| Salacia chinensis |
| Salacia reticulata |
| Centella asiatica |

| LIST OF ANTI-HYPERLIPIDEMIC MEDICINAL PLANTS |
|---|
| **Latin names** |
| Allium satinum |
| Commiphora mukul |
| Terminalia arjuna |
| Acorus calamus |
| Heloptelea integrifolia |
| Rubia Cordifolia |
| Smilax china |
| Sphearanthus indicus |
| Colchicum luteum |

| LIST OF ANTI-INFLAMMATORY MEDICINAL PLANTS |
|---|
| **Latin names** |
| Boswellia serrata |
| Withania somnifera |
| Ricinus communis |
| Pluchea lanceolata |
| Zingiber officinale |
| Glycyrrhiza glabra |
| Vitex negundo |
| Aloe vera |
| Tribulus terrestris |
| Ocimum sanctum |
| Crataeva nurvala |
| Acorus calamus |
| Commiphora mukul |
| Tinospora cordifolia |
| Rubia cordifolia |
| Cyperus rotundas |
| Moringa oleifera |
| Celastrus paniculatua |
| Aegle marmelos |
| Stereospermum suaveolens |
| Gmelina arborea |
| Oroxylum indicum |
| Premna mucronata |
| Solanum indicum |
| Solanum xanthocarpum |
| Desmodium gyrens |
| Uraria picta |

| LIST OF GASTRO-INTESTINAL AGENTS |
|---|
| **Latin names** |
| Cyperus rotundus |
| Carica papaya |
| Zingiber officinale |
| Curcuma longa |
| Menth spicata |
| Anethum sowa |
| Foeniculum vulgare |
| Plumbago zeylanica |
| Piper nigrum |
| Cuminum cyminum |
| Carum bulbocastom |
| Foerula asafetida |
| Punica granatum |
| Zingiber officinale |
| Garcinia indica |
| Oxalis corniculata |
| Aconitum heterophyllum |

### Description of Figures

### Figure 1

Method of preparation, process flow chart

### Figure 2

Gymnemic acid

### References

1. WHO, Fact sheet No. 236, Revised September 2002
2. Pharmacology, H.P. Rang, M.M. Dale, 1991
3. Drug information for the Health Care Professional, The United States Pharmacopoeial Convention, Inc., 1990
4. Martindale, The Extra Pharmacopoeia, James E.F. Reynolds, 1989
5. The Principles and Practice of Medicine, Harvey, Johns, Mc Kusick, Owens, Ross, 1988
6. Review of Medical Pharmacology, F.H. Meyers, Ernest Javetz, Alan Goldfien, 1980
7. The Pharmacological basis of Therapeutics, Louis S. Goodman, Alfred Gilman, 1965
8. R Yeganarayan, AP Saraf, JH Balwani, Comparison of anti-inflammatory activity of various extracts of Curcuma Longa. Indian J Med Res, 64 (1976) 601-608.

## Claims

1. A composition comprising:
a) an anti hyperglycaemic agent
b) an anti inflammatory agent
c) an anti hyperlipidemic agent
d) an anti oxidant agent
e) a gastro-intestinal agent.

2. A composition according to claim 1, wherein at least one of said agents is derived from a plant.

3. A composition according to claim 1, wherein at least one of said agents is a synthetic compound.

4. A composition according to claim 2, wherein said at least one agent is the anti hyperglycaemic agent.

5. A composition according to claim 4, wherein the anti hyperglycemic agent is derived from one or more plants from the group of Gymnema Sylvestre, Momordica Charantia, Trigonella Foenum Graecum, Curcuma Longa, Holarrhena Antidysenterica, Emblica Officinalis, Terminalia Belerica and Terminalia Chebula or a plant with comparable pharmacological action.

6. A composition according to claim 2, wherein said at least one agent is the anti inflammatory agent.

7. A composition according to claim 6, wherein the anti inflammatory agent is derived from one or more plants from the group of Curcuma Longa, Emblica Officinalis and Momordica Charantia or a plant with comparable pharmacological action.

8. A composition according to claim 2, wherein said at least one agent is the anti hyperlipidemic agent.

9. A composition according to claim 8, wherein the anti hyperlipidemic agent is derived from one or more plants from the group of Emblica Officinalis, Trigonella Foenum Graecum, Terminalia Chebula, Terminalia Belerica, Curcuma Longa, Gymnema Sylvestre and Momordica Charantia or a plant with comparable pharmacological action.

10. A composition according to claim 2, wherein said at least one agent is the anti oxidant agent.

11. A composition according to claim 10, wherein the anti oxidant agent is derived from one or more plants from the group of Curcuma Longa, Emblica Officinalis, Terminalia Belerica and Terminalia Chebula or a plant with comparable pharmacological action.

12. A composition according to claim 2, wherein said at least one agent is the gastro-intestinal agent.

13. A composition according to claim 12, wherein the gastro-intestinal agent is derived from one or more plants from the group Holarrhena Antidysenterica and Curcuma Longa or a plant with comparable pharmacological action.

14. A composition according to any one of claims 1 to 13, wherein the agents are present in the following amounts, based on total composition and based on primary pharmacological action
a) 10 - 50 weight % of the anti hyperglycaemic agent
b) 10 - 30 weight % of the anti inflammatory agent
c) 10 - 50 weight % of the anti hyperlipidemic agent
d) 10 - 30 weight % of the anti oxidant agent
e) 0.05 - 10 weight % of the gastro-intestinal agent

15. A composition according to any one of claims 1, 2 and 4-14, comprising a part or an extract from Curcuma Longa, Emblica Officinalis, Gymnema Sylvestre, Holarrhena Antidysenterica, Momordica Charantia, Terminalia Belerica, Terminalia Chebula and Trigonella Foenum Graecum or a plant with comparable pharmacological action if compared to the mentioned plants.

16. A composition according to claim 15 which comprises:
a) 0.05 - 25 weight % of Curcuma Longa
b) 0.05 - 25 weight % of Emblica Officinalis
c) 0.05 - 25 weight % of Gymnema Sylvestre
d) 0.05 - 25 weight % of Holarrhena Antidysenterica
e) 0.05 - 25 weight % of Momordica Charantia
f) 0.05 - 25 weight % of Terminalia Belerica
g) 0.05 - 25 weight % of Terminalia Chebula
h) 0.05 - 25 weight % of Trigonella Foenum Graecum

17. A pharmaceutical composition comprising a composition as claimed in any one of claims 1 to 16.

18. Use of a composition as claimed in any one of claims 1 to 16 in the preparation of a medicament for the treatment of diabetes.

19. A method for treating a person in need thereof comprising administering an effective of amount of a composition according to any one of claims to said person.

20. A method according to claim 19, wherein said person is suffering from (symptoms associated with) diabetes.
